# EUROPEAN PATENT SPECIFICATION

(11) **EP 2 882 291 B1**
(45) Date of publication and mention of the grant of the patent: **27.09.2017**
(21) Application number: 12882796.1
(22) Date of filing: 29.10.2012
(51) Int. Cl.: C07C 231/10, C07C 209/50, C07C 233/06, C07C 211/38

(54) **AN IMPROVED PROCESS FOR THE PREPARATION OF MEMANTINE HYDROCHLORIDE**
VERBESSERTES VERFAHREN ZUR HERSTELLUNG VON MEMANTIN-HYDROCHLORID
PROCÉDÉ AMÉLIORÉ POUR LA PRÉPARATION DE CHLORHYDRATE DE MÉMANTINE

(30) Priority: 07.08.2012 IN 2259MU2012
(43) Date of publication of application: 17.06.2015
(73) Proprietor: ZCL Chemicals Ltd., 400 059 Mumbia, Maharashtra (IN)
(72) Inventor: AGARWAL, Nand Lal, 393002 Gujarat (IN); MISTRI, Pranav Popatlal, 393002 Gujarat (IN); PATEL, Nitin Maganbhai, 393002 Gujarat (IN)
(74) Representative: Gislon, Gabriele
(86) International application number: PCT/IN2012/000709
(87) International publication number: WO 2014/024202

(56) References cited:
- WO-A1-2007/126886
- WO-A1-2008/040560
- WO-A2-2009/057140
- WO-A2-2009/057140
- US-A1- 2006 258 885
- US-A1- 2010 204 470

## Description

### FIELD OF THE INVENTION

The present invention relates to an improved process for the preparation of Memantine hydrochloride of formula (1).

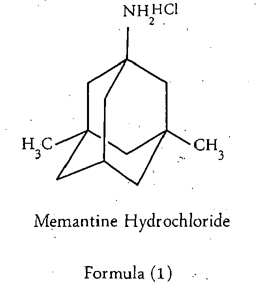

Memantine hydrochloride is chemically known as 1-amino-3,5-dimethyladamantane hydrochloride used in the treatment of Alzheimer's and Parkinson's disease.

### BACKGROUND OF THE INVENTION

Prior art has suggested references for the preparation of Memantine hydrochloride.

United State Patent 3,391,142 discloses a process for the preparation of Memantine Hydrochloride wherein 1,3-dimethyladamantane is reacted with bromine and afforded 1-bromo-3,5-dimethyladamantane. 1-bromo-3,5-dimethyl Adamantane is reacted with sulphuric acid in acetonitrile gives 1-acetamido-3,5-dimethyladamantane. 1-acetamido-3,5-dimethyladamantane is then taken for hydrolysis with sodium hydroxide in diethylene glycol gives Memantine which is then converted into its Hydrochloride salt by treating with anhydrous hydrogen chloride to obtain Memantine Hydrochloride of formula (1). Memantine Hydrochloride is crystallized from a mixture of alcohol and ether to give pure Memantine Hydrochloride.

There are several disadvantages of the said process. Bromination is reaction is carried out at reflux temperature that leads to toxic vapors further tertiary bromide obtained from a bromination reaction is a hazardous intermediate and it is difficult to handle such an intermediate and one needs to take utmost care in storing such a hazardous chemical. Diethylene glycol used in the reaction is hazardous and poisonous solvent. Diethylene glycol when heated with sodium hydroxide produces hydrogen gas which on the larger scale may cause a serious accident. Further, in the final step of the purification ether which is flammable in nature, difficult to handle on a large scale or commercial scale and it is not an industrially viable process.

According to the process disclosed in the PCT International patent application WO2010067252A1, 1,3-dimethyladamantane and bromine reacted in presence of catalytic amount of HBr in acetic acid and afforded 1-Bromo-3,5-dimethyladamantane and converted into 1-Acetamido-3,5-Dimethyl Adamantane in presence of Acetonitrile and Sulphuric acid. Later on 1-Acetamido-3,5-dimethyladamantane is converted into Memantine free base or salt. Patent is also having the disadvantages which are similar to the disadvantages of United State Patent 3,391,142.

PCT International patent application WO2005023753 discloses a process for the preparation of Memantine hydrochloride by using bromine to get the 1-bromo-3, 5-dimethyladamantane which is then reacted with urea/formic acid to give 1-acetamido analogue & then hydrolysis & acidification with HCl to obtain Memantine hydrochloride.

Another PCT International patent application WO2005062724 discloses a process for the preparation of Memantine Hydrochloride wherein bromination of 1,3-dimethyladamantane, followed by hydrolysis to yield 1-Hydroxy-3,5-dimethyladamantane, which is treated with Acetonitrile in presence of an acid (e.g. Sulphuric acid) to yield the 1-acetamido-3,5-dimethyladamantane. The 1-acetamido-3,5-dimethyladamantane compound is hydrolyzed in the presence of acid or base to yield the Amino-Adamantane derivative like Memantine.

EP1674446 discloses a process for the preparation of Memantine Hydrochloride wherein 1-bromo-3,5-dimethyladamantane is reacted with urea in 80% formic acid at 80°C followed by acid hydrolysis to yield crude Memantine which is further converted to Memantine hydrochloride in ethanolic HCl. The crude product is further crystallized in alcohol, ketone, water or a mixture thereof to yield pure memantine HCl.

The other PCT International patent application WO2009153806 describes a process for the preparation of Memantine Hydrochloride wherein 1,3-dimethyladamantane is reacted with Acetonitrile and sulfuric acid to give 1-Acetamido-3,5-dimethyl-adamantane.1-Acetamido-3,5-dimethyladamantane as obtained is with base selected from bases such as NaOH, KOH, Lithium hydroxide, Barium carbonate or Cesium carbonate in high boiling solvent selected from the solvents like monoethylene glycol, polyethylene glycol, Diethylene glycol, or hazardous solvent such as dichloromethane, dichloroethane, carbon tetrachloride or chloroform to give Memantine. Memantine is reacted with alcoholic HCl (methanol/ethanol/isopropanol/butanol) in a solvent selected from solvents like MIBK, cyclohexanone, cyclopentanone or acetone to yield Memantine HCl salt. Purification is required to obtain pure Memantine Hydrochloride.

PCT international application WO2009057140 discloses a process for the preparation of Memantine Hydrochloride wherein 1,3-dimethyladamantane is reacted with Acetonitrile in presence of sulphuric acid at a temperature of about 50°C-70°C for period of time about 2 to 15hours to provide 1-acetamido-3,5-dimethyladamantane in absence of any solvent. Hydrolyzing 1-acetamido-3,5-dimethyladamantane with a base like Sodium Hydroxide in high boiling solvent such as polyethylene glycol at a temperature of about 120°C-180°C for a period of time about 2 to 25hours to give Memantine which on subsequent treatment with hydrochloric acid in isopropyl alcohol followed by adding ester solvent to obtain Memantine hydrochloride.

Above processes are having several disadvantages.

Process as disclosed in the PCT Patent Application WO2009153806 involves use of carbinol during reaction, number of operation in isolation of 1-acetamido-3,5-dimethyladamantane such as (i) extraction with solvent, (ii) washing with alkali, (iii) evaporation of solvent and (iv) adding another solvent for isolation, lengthy process that is carried out at a high temperature & harsh reaction condition and generates high effluent during the process. Also Memantine Hydrochloride as produced with the said process requires purification in appropriate solvent to obtain desired quality of Memantine hydrochloride that leads to the loss in yield. Hence the process fails to prove its capability as industrially viable and environment friendly process.

PCT Application WO2009057140 process involves the use of several solvents during manufacturing of 1-acetamido-3,5-dimethyladamantane which is a very tedious, lengthy and complex process. The patent has disclosed hydrolysis reaction using Polyethylene glycol as solvent especially which is a high boiling and water miscible solvent. Polyethylene glycol used in the reaction is hazardous and poisonous solvent. Polyethylene glycol when heated with base like sodium hydroxide produces hydrogen gas which on the larger scale may cause a serious accident. Use of Polyethylene glycol as a reaction solvent may increase the effluent in excess amount which is again a critical question to disposal the same. Hence this process is not suitable for,the industrial purpose.

Further, the above two patent applications i.e. (i) WO2009153806 and (ii) WO2009057140 disclose a process for the preparation of Memantine hydrochloride give less overall yield.

Still there is a need to provide environment friendly, commercially viable process for the preparation of Memantine Hydrochloride that involves fewer operations in the preparation of Memantine hydrochloride of formula (1).

The present invention provides environment and plant friendly process which reduces process operation stages and thereby saving the manufacturing cost, manpower, time cycles and can avoid generation of unwanted excessive effluent.

### OBJECTS OF THE INVENTION

Therefore, the main object of the present invention is to overcome the above mentioned problems.

The other object of the present invention is to provide an improved process for the preparation of Memantine Hydrochloride of formula (1) which is economical, viable on a commercial scale, plant friendly and uses less equipments and need not required any purification.

There is also an object of the present invention to provide an improved process for the preparation of Memantine Hydrochloride of formula (1) having more than 99.9% purity comprises steps:
a) reacting 1,3-dimethyladamantane compound of formula (2) with acetonitrile in presence of sulphuric acid at a temperature between 25°C-45°C in absence of solvent to give 1-acetamido-3,5-dimethyladamantane of formula (3);
b) isolating 1-acetamido-3,5-dimethyladamantane compound of formula (3) with the addition of water in the reaction mass of step-(a);
c) hydrolyzing 1-acetamido-3,5-dimethyladamantane of formula (3) with base in presence of aqueous iso-butanol at a temperature 110°C-140°C to give Memantine free base of formula (4);
d) in-situ treating Memantine free base of formula (4) with hydrochloric acid at a temperature of 5°C to 35°C and distilling out aqueous iso-butanol completely and adding ester solvent into it to yield pure Memantine hydrochloride of formula (1).

### DETAILED DESCRIPTION OF THE INVENTION

Accordingly the present invention provides an improved process for the preparation of Memantine Hydrochloride of formula (1) which is economical, viable on a commercial scale; plant friendly and uses less equipments and need not required any purification.

The process as described in the present invention gives overall very good yield and purity.

The present invention schematically represented by the following scheme-1.

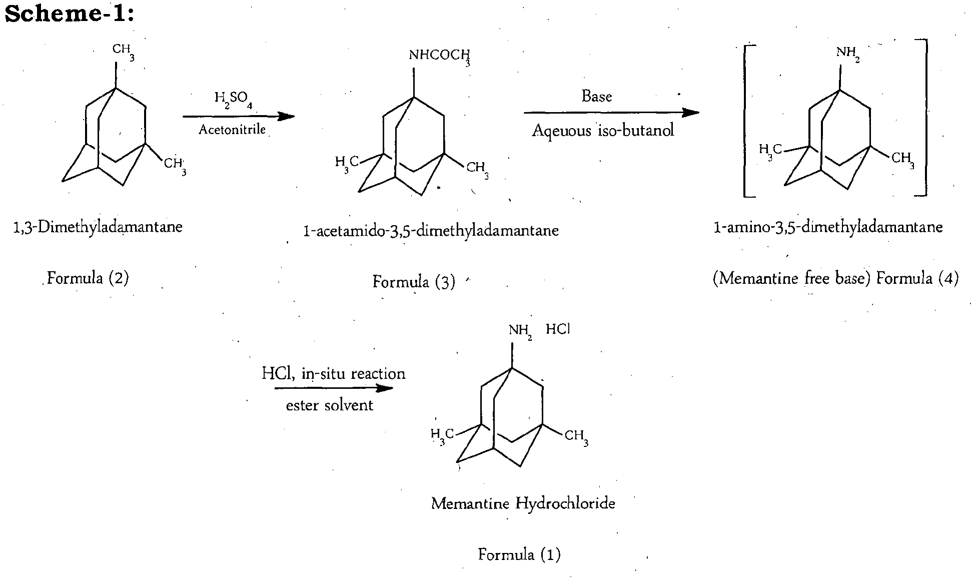

In a preferred embodiment, an improved process for the preparation of Memantine Hydrochloride of formula (1) having more than 99.9% purity comprises the steps:
a) reacting 1,3-dimethyladamantane compound of formula (2) with Acetonitrile in presence of sulphuric acid at a temperature between 25°C-45°C in absence of solvent to give 1-acetamido-3,5-dimethyladamantane of formula (3);
b) isolating 1-acetamido-3,5-dimethyladamantane compound of formula (3) with the addition of water in the reaction mass of step-(a);
c) hydrolyzing 1-acetamido-3,5-dimethyladamantane of formula (3) with base in presence of aqueous iso-butanol at a temperature 110°C-140°C to give Memantine free base of formula (4);
d) in-situ treating Memantine free base of formula (4) with hydrochloric acid at a temperature of 5°C to 35°C and distilling out aqueous iso-butanol completely and adding ester solvent into it to yield pure Memantine hydrochloride of formula (1).

According to the step-(a), 1,3-dimethyladamantane of formula (2) is reacted with Acetonitrile in presence of sulphuric acid at a temperature between 25°C-45°C in absence of solvent to produce 1-acetamido-3,5-dimethyladamantane of formula (3) in a reaction mass.

Acetonitrile used in the reaction is consumed in a quantity of 2 volume to 5 volume compared to 1,3-dimethyladamantane of formula (2) while sulphuric acid used in the reaction is consumed in a quantity of 5 volume to 10 volume compared to 1,3-dimethyladamantane of formula (2).

In step-(b), 1-acetamido -3,5-dimethyladamantane of formula (3) produced in the reaction mass is isolated by adding water into the reaction mass. Water content of the isolated product is varied between 0.1%w/w to 24%w/w.

Isolation of 1-acetamido-3,5-dimethyladamantane of formula (3) from the reaction mass may be done through any of the method as described here. Either water in a volume between 18v to 26v compared to the weight of 1,3-dimethyladamantane of formula (2) is added in the reaction mass at a temperature 0°C-40°C and stirred the reaction mass for 30 minutes and filtered the product and washed it with water to give 1-acetamido-3,5-dimethyladamantane of formula (3).
Or

Reaction mass is cooled to and it is quenched in water (water quantity is between 18v to 26v compared to the weight of 1,3-dimethyladamantane of formula (2)) under stirring at a temperature 0°C-40°C and stirred the reaction mass for 30 minutes, filtered the product and washed it with water to give 1-acetamido-3,5-dimethyladamantane of formula (3).

In step-(c),1-acetamido-3,5-dimethyladamantane of formula (3) as obtained in step-(b) is hydrolyzed using base in aqueous iso-butanol at a temperature between 110°C-140°C to give Memantine free base of formula (4) in reaction mass. Normally the reaction is completed in between 2-40hrs, preferably in 2 to 24hrs.
It is to be noted that the 1-acetamido-3,5-dimethyladamantane of formula (3) as obtained in step-(b) is isolated with addition of water and it can be used in step-(c) with or without drying completely. Water content of the reaction mass of step-(c) is maintained between 0.1%w/w to 10%w/w.

It is also to be noted that maintaining the water content of reaction of step-(c) between 0.1%w/w to 10%w/w helps in reducing the impurity profile and improve the appearance of the final product i.e. Memantine Hydrochloride of formula (1).
On the other side water content more than 10%w/w may increase reaction time and the starting material i.e. 1-acetamido-3,5-dimethyladamantane of formula (3) remains unreacted in the reaction thus water content more than 10%w/w in the reaction of step-(c) reduces the yield of the final product i.e. Memantine Hydrochloride of formula (1).

Base used in the reaction is selected from bases such as sodium hydroxide, potassium hydroxide, sodium tert-butoxide, sodium butoxide, sodium ethoxide, sodium methoxide, potassium tert-butoxide, potassium butoxide, potassium ethoxide or potassium methoxide, preferably sodium hydroxide or potassium hydroxide.

Base quantity used in the reaction may be varied between 4 to 12moles/mole of 1-acetamido-3,5-dimethyladamantane, preferably 4 to 6 moles/mole of 1-acetamido-3,5-dimethyladamantane.

The reaction mass of step-(c) is taken for work up. Reaction mass is cooled to 60°C-70°C, water is added into it under stirring and settled the reaction mass till two layers are formed. Organic layer is separated and washed with water. Organic layer contains Memantine free base of formula (4) which is then proceed for the next step-(d).

In step-(d), organic layer as obtained in step-(c) is reacted with concentrated hydrochloric acid to give Memantine Hydrochloride of formula (1).

During the reaction, temperature is maintained between 5°C-35°C, preferably 10°C-25°C. Concentrated hydrochloric acid used in the reaction is selected from hydrochloride gas, aqueous hydrochloric acid having concentration between 25v/w-35%v/w or HCl gas dissolved in iso-butanol solvent.

The said reaction mass is distilled under reduced pressure at below 75°C to remove water and iso-butanol completely. Residue obtained after distillation is cooled to 30°C-40°C,ester solvent is added in the reaction mass, further cooled to 0°C-10°C, stirred for 1 hr and filtered to get pure Memantine Hydrochloride of formula (1).

Ester solvent used in the reaction is selected from ester solvents like n-propyl acetate, isopropyl acetate, isobutyl acetate or n-butyl acetate or mixtures thereof.

Memantine Hydrochloride obtained by the above process is more than 99.9% pure and is an acceptable pharma product and no further purification is required.

The present invention demonstrated examples cited below, which are provided as illustration only and therefore should not be construed as limitation of the possible and future invention.

### EXAMPLES

### Examples-1: Preparation of 1-acetamido-3,5-dimethyladamantane of formula (3):

Into 1,3-dimethyladamantane (10grams), sulphuric acid (56ml) added and slowly acetonitrile (37.5ml) was added at 25°C-45°C. The reaction mixture was stirred 25°C-45°C. The reaction mixture was quenched into water. Later filtration followed by water washing and drying to get title compound.
Yield: 12.5 grams
Purity by GC: 99.52%
Water content (moisture): 0.5%w/w

### Examples-2: Preparation of 1-acetamido-3,5-dimethyladamantane of formula (3):

Into 1,3-dimethyladamantane (10grams), sulphuric acid (56ml) added and slowly acetonitrile (37.5ml) was added at 25°C-45°C. The reaction mixture was stirred at 25°C-45°C. The water was added in reaction mixture at 20°C-30°C. Then filtration followed by water washing to get the wet cake of title compound.
Yield: 15.06grams wet cake (12.8grams on dried basis)
Purity by GC: 99.50%
Water content (moisture): 15%w/w

### Examples-3: Preparation of Memantine hydrochloride (1) from 1-acetamido-3,5-dimethyladamantane (3):

A mixture of 1-acetamido-3,5-dimethyladamantane (10grams), iso-butanol (40ml) and potassium hydroxide (15.8gram) was heated to 110°C-140°C and then stirred for 20hours at the same temperature. Reaction mixture was cooled to 65°C-75°C. Added water into the reaction mixture and stirred for 15 minutes. The organic and aqueous layers were separated. The organic layer pH was adjusted 8.5 to 10.5 with adding a base and stirred it for 15minutes. The organic and aqueous layers were separated. Into organic layer conc. Hydrochloric acid was added and stirred for 15minutes. Iso-butanol and water was distilled off under reduced pressure below 75°C. In obtained residue 40ml n-butyl acetate was added and reaction mixture was heated to 45°C and stirred for 30minutes. The reaction mixture was cooled to 0°C-5°C. The solid was filtered, dried at 50°C for 12hours to get the title compound.
Yield: 9.0grams
Purity by GC: 99.96%

### Examples-4: Preparation of Memantine hydrochloride (1) from 1-acetamido-3, 5-dimethyladamantane (3):

The title compound is prepared in same manner of example-3, wherein 9% aqueous iso-butanol was used as a solvent in place of iso-butanol.
Yield: 9.0grams
Purity by GC: 99.97%

### Examples-5: Preparation of Memantine hydrochloride (1) from 1-acetamido-3,5-dimethyladamantane (3):

The title compound is prepared in analogues manner of example-2, wherein iso-butyl acetate was used as a solvent in place of n-butyl acetate.
Yield: 8.9grams
Purity by GC: 99.95%

## Claims

1. An improved process for the preparation of Memantine Hydrochloride of formula (1) having more than 99.9% purity comprises the steps:
a) reacting 1,3-dimethyladamantane compound of formula (2) with Acetonitrile in presence of sulphuric acid at a temperature between 25°C-45°C in absence of solvent to give 1-acetamido-3,5-dimethyladamantane of formula (3);
b) isolating 1-acetamido-3,5-dimethyladamantane compound of formula (3) with the addition of water in the reaction mass of step-(a);
c) hydrolyzing 1-acetamido-3,5-dimethyladamantane of formula (3) with base in presence of aqueous iso-butanol at a temperature 110°C-140°C to give Memantine free base of formula (4);
d) in-situ treating Memantine free base of formula (4) with hydrochloric acid at a temperature of 5°C to 35°C and distilling out aqueous iso-butanol completely and adding ester solvent into it to yield pure Memantine Hydrochloride of formula (1).

2. An improved process for the preparation of Memantine Hydrochloride as claimed in claim (1) wherein in step-(a), acetonitrile is used in a quantity between 2 volumes to 5 volumes compared to 1,3-dimethyladamantane of formula (2).

3. An improved process for the preparation of Memantine Hydrochloride as claimed in claim (1) wherein in step-(a), sulphuric acid is used in a quantity between 5 volumes to 10 volumes compared to 1,3-dimethyladamantane of formula (2).

4. An improved process for the preparation of Memantine Hydrochloride as claimed in claim (1) wherein in step-(b), water is added in the reaction mass at a temperature between 0°C to 40°C.

5. An improved process for the preparation of Memantine Hydrochloride as claimed in claim (1) wherein in step-(b), wherein 1-acetamido-3,5-dimethyladamantane of formula (3) is isolated using filtration.

6. An improved process for the preparation of Memantine Hydrochloride as claimed in claim (1) wherein in step-(b), water content of the isolated product 1-acetamido-3,5-dimethyladamantane of formula (3) is between 0.1%w/w to 24%w/w.

7. An improved process for the preparation of Memantine Hydrochloride as claimed in claim (1) wherein in step-(c), base is selected from the bases like sodium hydroxide, potassium hydroxide, sodium tert-butoxide, sodium butoxide, sodium iso-butoxide, sodium ethoxide, sodium methoxide, potassium tert-butoxide, potassium butoxide, potassium iso-butoxide, potassium ethoxide, potassium methoxide, preferably sodium hydroxide or potassium hydroxide.

8. An improved process for the preparation of Memantine Hydrochloride as claimed in claim (1) wherein in step-(c), wherein water content of aqueous iso-butanol is maintained between 0.1%w/w to 10%w/w.

9. An improved process for the preparation of Memantine Hydrochloride as claimed in claim (1) wherein in step-(d), hydrochloric acid used in the reaction is selected from hydrochloride gas, aqueous hydrochloric acid having concentration between 25%w/w-35%w/w or HCl gas dissolved in iso-butanol solvent.

10. An improved process for the preparation of Memantine Hydrochloride as claimed in claim (1) wherein in step-(d), ester solvent is selected from n-propyl acetate, isopropyl acetate, isobutyl acetate or n-butyl acetate or mixtures thereof.

## Patentansprüche

1. Ein verbessertes Verfahren zur Herstellung von Memantin-Hydrochlorid der Formel (1) mit mehr als 99,9% Reinheit umfassend die Schritte:
a) Umsetzung einer 1,3-Dimethyladamantanverbindung der Formel (2) mit Acetonitril in Gegenwart von Schwefelsäure bei einer Temperatur zwischen 25 °C und 45 °C in Abwesenheit von Lösungsmittel, um 1-Acetamido-3,5-dimethyladamantan der Formel (3) zu ergeben;
b) Isolierung von 1-Acetamido-3,5-dimethyladamantan-Verbindung der Formel (3) unter Zugabe von Wasser in der Reaktionsmasse von Schritt (a);
c) Hydrolysieren von 1-Acetamido-3,5-dimethyladamantan der Formel (3) mit Base in Gegenwart von wässrigem Isobutanol bei einer Temperatur von 110 °C bis 140 °C, um eine Memantin-freie Base der Formel (4) zu ergeben;
d) In-situ-Behandlung der Memantin-freien Base der Formel (4) mit Salzsäure bei einer Temperatur von 5 °C bis 35 °C und vollständigem Abdestillieren von wässrigem Isobutanol und Zugabe von Esterlösungsmittel dazu, um reines Memantin-Hydrochlorid der Formel (1) zu ergeben.

2. Verbessertes Verfahren zur Herstellung von Memantin-Hydrochlorid nach Anspruch 1, wobei in Schritt (a) Acetonitril in einer Menge von zwischen 2 Volumina und 5 Volumina im Vergleich zu 1,3-Dimethyladamantan der Formel (2) verwendet wird.

3. Verbessertes Verfahren zur Herstellung von Memantin-Hydrochlorid nach Anspruch 1, **dadurch gekennzeichnet, dass** in Schritt (a) Schwefelsäure in einer Menge zwischen 5 Volumina und 10 Volumina im Vergleich zu 1,3-Dimethyladamantan der Formel (2) verwendet wird.

4. Verbessertes Verfahren zur Herstellung von Memantin-Hydrochlorid nach Anspruch 1, wobei in Schritt (b) Wasser in der Reaktionsmasse bei einer Temperatur zwischen 0 °C und 40 °C zugegeben wird.

5. Verbessertes Verfahren zur Herstellung von Memantin-Hydrochlorid nach Anspruch 1, wobei in Schritt (b) 1-Acetamido-3,5-dimethyladamantan der Formel (3) unter Verwendung von Filtration isoliert wird.

6. Verbessertes Verfahren zur Herstellung von Memantin-Hydrochlorid nach Anspruch 1, wobei in Schritt (b) der Wassergehalt des isolierten Produkts 1-Acetamido-3,5-dimethyladamantan der Formel (3) zwischen 0,1 Gew.-% und 24 Gew.-% ist.

7. Verbessertes Verfahren zur Herstellung von Memantin-Hydrochlorid nach Anspruch 1, wobei in Schritt (c) die Base ausgewählt ist aus den Basen wie Natriumhydroxid, Kaliumhydroxid, Natrium-tert-butoxid, Natriumbutoxid, Natriumisobutoxid, Natriumethoxid, Natriummethoxid, Kalium-tert-butoxid, Kaliumbutoxid, Kaliumisobutoxid, Kaliumethoxid, Kaliummethoxid, vorzugsweise Natriumhydroxid oder Kaliumhydroxid.

8. Verbessertes Verfahren zur Herstellung von Memantin-Hydrochlorid nach Anspruch 1, wobei in Schritt (c) der Wassergehalt an wässrigem Isobutanol zwischen 0,1 Gew.-% und 10 Gew.-% gehalten wird.

9. Verbessertes Verfahren zur Herstellung von Memantin-Hydrochlorid nach Anspruch 1, wobei in Schritt (d) die in der Reaktion verwendete Salzsäure ausgewählt ist aus Hydrochlorid-Gas, wässriger Salzsäure mit einer Konzentration zwischen 25 Gew.-% und 35 Gew.-% oder HCl-Gas, gelöst in iso-Butanol-Lösungsmittel.

10. Verbessertes Verfahren zur Herstellung von Memantin-Hydrochlorid nach Anspruch 1, wobei in Schritt (d) das Esterlösungsmittel ausgewählt ist aus n-Propylacetat, Isopropylacetat, Isobutylacetat oder n-Butylacetat oder Gemischen davon.

## Revendications

1. Procédé amélioré de préparation de chlorhydrate de mémantine de formule (1) ayant une pureté supérieure à 99,9 % comprenant les étapes suivantes :
a) mise en réaction d'un composé 1,3-diméthyladamantane de formule (2) avec de l'acétonitrile en présence d'acide sulfurique à une température comprise entre 25 °C et 45 °C en l'absence de solvant pour obtenir le 1-acétamido-3,5-diméthyladamantane de formule (3) ;
b) isolement du composé 1-acétamido-3,5-diméthyladamantane de formule (3) par ajout d'eau dans la masse réactionnelle de l'étape (a) ;
c) hydrolyse du 1-acétamido-3,5-diméthyladamantane de formule (3) avec une base en présence d'iso-butanol aqueux à une température de 110 °C à 140 °C pour donner une base libre de mémantine de formule (4) ;
d) traitement in situ de la base libre de mémantine de formule (4) avec de l'acide chlorhydrique à une température de 5 °C à 35 °C et distillation complète de l'iso-butanol avant ajout de solvant ester à cela pour donner du chlorhydrate de mémantine pur de formule (1).

2. Procédé amélioré de préparation de chlorhydrate de mémantine selon la revendication (1), dans lequel à l'étape (a), l'acétonitrile est utilisé en une quantité comprise entre 2 volumes et 5 volumes comparé au 1,3-diméthyladamantane de formule (2).

3. Procédé amélioré de préparation de chlorhydrate de mémantine selon la revendication (1), dans lequel à l'étape (a), l'acide sulfurique est utilisé en une quantité comprise entre 5 volumes et 10 volumes comparé au 1,3-diméthyladamantane de formule (2).

4. Procédé amélioré de préparation de chlorhydrate de mémantine selon la revendication (1), dans lequel à l'étape (b), de l'eau est ajoutée à la masse réactionnelle à une température comprise entre 0 °C et 40 °C.

5. Procédé amélioré de préparation de chlorhydrate de mémantine selon la revendication (1), dans lequel à l'étape (b), le 1-acétamido-3,5-diméthyladamantane de formule (3) est isolé en utilisant la filtration.

6. Procédé amélioré de préparation de chlorhydrate de mémantine selon la revendication (1), dans lequel à l'étape (b), la teneur en eau du produit isolé 1-acétamido-3,5-diméthyladamantane de formule (3) est comprise entre 1 % p/p et 24 % p/p.

7. Procédé amélioré de préparation de chlorhydrate de mémantine selon la revendication (1), dans lequel à l'étape (c), la base est sélectionnée parmi les bases de type hydroxyde de sodium, hydroxyde de potassium, tert-butoxyde de sodium, butoxyde de sodium, iso-butoxyde de sodium, éthoxyde de sodium, méthoxyde de sodium, tert-butoxyde de potassium, butoxyde de potassium, iso-butoxyde de potassium, éthoxyde de potassium, méthoxyde de potassium, de préférence hydroxyde de sodium ou hydroxyde de potassium.

8. Procédé amélioré de préparation de chlorhydrate de mémantine selon la revendication (1), dans lequel à l'étape (c), la teneur en eau de l'iso-butanol aqueux est maintenue entre 0,1 % p/p et 10 % p/p.

9. Procédé amélioré de préparation de chlorhydrate de mémantine selon la revendication (1), dans lequel à l'étape (d), l'acide chlorhydrique utilisé dans la réaction est sélectionné parmi le gaz de chlorhydrate, l'acide chlorhydrique aqueux ayant une concentration comprise entre 25 % p/p et 35 % p/p ou le gaz de HCl dissous dans un solvant iso-butanol.

10. Procédé amélioré de préparation de chlorhydrate de mémantine selon la revendication (1), dans lequel à l'étape (d), le solvant ester est sélectionné parmi l'acétate de n-propyle, l'acétate d'isopropyle, l'acétate d'isobutyle ou l'acétate de n-butyle ou les mélanges de ceux-ci.
